# EUROPEAN PATENT SPECIFICATION

(11) **EP 4 227 663 B1**
(45) Date of publication and mention of the grant of the patent: **10.07.2024**
(21) Application number: 22156581.5
(22) Date of filing: 14.02.2022
(51) Int. Cl.: G01N 11/00, G01N 33/44

(54) **METHOD AND APPARATUS FOR ADAPTING OPERATION SETTINGS FOR A FREQUENCY SWEEP MEASUREMENT OF A POLYMER PRODUCT**
VERFAHREN UND VORRICHTUNG ZUR ANPASSUNG VON BETRIEBSEINSTELLUNGEN FÜR EINE FREQUENZABTASTUNGSMESSUNG EINES POLYMERPRODUKTS
PROCÉDÉ ET APPAREIL D'ADAPTATION DES PARAMÈTRES DE FONCTIONNEMENT POUR UNE MESURE PAR BALAYAGE DE FRÉQUENCE D'UN PRODUIT POLYMÈRE

(43) Date of publication of application: 16.08.2023
(73) Proprietor: Borealis AG, 1020 Vienna (AT)
(72) Inventor: Touloupidis, Vasileios, 4021 Linz (AT); Knogler, Bernhard, 4021 Linz (AT); Barroso, Vitor, 4021 Linz (AT); Jarl, Anna-Karin, 444 86 Stenungsund (SE); Kaufmann, Christoph, 84489 Burghausen (DE); Carlsson, Niclas, 444 86 Stenungsund (SE); Skalen, Staffan, 444 86 Stenungsund (SE)
(74) Representative: Maiwald GmbH

(56) References cited:
- US-A1- 2012 312 073
- US-A1- 2018 045 630
- US-A1- 2021 277 153

## Description

### FIELD OF THE INVENTION

The present invention relates to methods for polymer rheological characterization via fast frequency sweep and in particular to a method and an apparatus for adapting operation settings for a frequency sweep measurement of a polymer product.

### BACKGROUND OF THE INVENTION

In order to monitor and control the properties of a polymer during production, reactor sample needs to be collected and characterized with a material- or parameter-characterizing method of choice at regular intervals, defining the manipulative moves.

The measurement feedback frequency, for classic common-use reactors typically every 4 to 6 hours, limits the selection of the characterization methods only to those that can provide results within this timeframe. For instance, the total measurement time includes sample HLZ: SQL
acquisition, sample preparation, measurement preparation, sample heating-up, and actual measurement. Furthermore, additional limitations connected to technical expertise level and plant resources further apply.

Ideally, the knowledge of the actual molecular weight distribution would be needed as it is the basis of all secondary properties (rheological, mechanical). In practice however, this is prohibitive for full-scale plant environment (high level resources needed in terms of expertise personnel, materials and high measurement time).

Document US 2012/312073 A1 discloses an air bubble detection system. Air bubbles may be characterized by choosing an optimum set of frequencies and then comparing a return signal from a sensor receiving those frequencies against an internal reference. The number of pulses that exceed the internal reference represents a width and may be counted. The width, as counted, may be correlated to bubble characteristics including volume.

Thus, there is a need to improve polymer rheological characterization as used in current polymer reactors.

### SUMMARY OF THE INVENTION

The foregoing and other objects are solved by the subject-matter of the present invention as defined by the independent claims. Further embodiments are defined by the dependent claims. According to a first aspect of the present invention, a method is provided, a method for adapting operation settings for a frequency sweep measurement of a polymer product.

The method comprises the step of providing at least one set of operation setting parameters comprising at least one frequency range for the frequency sweep measurement of the polymer product, the at least one frequency range comprising a plurality of specific measurement frequencies.

The method comprises the step of optimizing the frequency range by deleting at least one measurement frequency of the plurality of specific measurement frequencies within a lower half of the frequency range resulting in a first optimized frequency range.

The method comprises the step of optimizing the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range.

In other words, the present invention advantageously provides a set of special operation settings for the frequency sweep measurement that leads to significant reduction of measurement time while the method accuracy remains at an adequate level for the needs of plant control.

Further, the present invention advantageously allows that the settings lead to significant reduction of the measurement time.

According to one exemplary embodiment of the present invention, after the step of optimizing the frequency range by deleting at least one measurement frequency, the first optimized frequency range is further optimized by equally distributing the remaining specific measurement frequencies within the lower half of the frequency range or within the complete frequency range.

According to one exemplary embodiment of the present invention, after the step of optimizing the first optimized frequency range, the second optimized frequency range is further optimized by equally distributing the remaining specific measurement frequencies within the subrange of the frequency range or within the complete frequency range.

According to one exemplary embodiment of the present invention, the method further comprises the step of validating a consistency of the second optimized frequency range by comparing a calculated complex viscosity value to a measured melt flow index value using the second optimized frequency range.

According to one exemplary embodiment of the present invention, the method further comprises the steps of applying the second optimized frequency range for quality control measurements of the polymer product.

According to one exemplary embodiment of the present invention, the method further comprises the steps of applying the second optimized frequency range for a currently employed melt flow index measurement. This step might be combined with the previously mentioned step of applying the second optimized frequency range for quality control measurements of the polymer product.

According to one exemplary embodiment of the present invention, the method further comprises the steps of generating the second optimized frequency range for a specific polymer grade of interest of the polymer product.

According to one exemplary embodiment of the present invention, by generating the second optimized frequency range for the specific polymer grade of interest of the polymer product, a frequency sweep fingerprint for the specific polymer grade is defined.

According to one exemplary embodiment of the present invention, the set of operation setting parameters comprises at least one shear rate.

According to a second aspect of the present invention, a computer program product is provided comprising computer-readable instructions which, when loaded and executed on processor, performs the method according to any one of the embodiments of the first aspect or the first aspect as such.

According to a third aspect of the present invention, an apparatus is provided, the apparatus configured for adapting operation settings for a frequency sweep measurement of a polymer product, the apparatus comprising a data memory and a processor.

The data memory is configured to provide at least one set of operation setting parameters comprising at least one frequency range for the frequency sweep measurement of the polymer product, the at least one frequency range comprising a plurality of specific measurement frequencies.

The processor is configured to optimize the frequency range by deleting at least one measurement frequency of the plurality of specific measurement frequencies within a lower half of the frequency range resulting in a first optimized frequency range.

The processor is configured to optimize the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range.

According to one embodiment of the present invention, the processor is further configured to optimize the first optimized frequency range by equally distributing the remaining specific measurement frequencies within the lower half of the frequency range or within the complete frequency range.

According to one embodiment of the present invention, the processor is further configured to optimize the second optimized frequency range by equally distributing the remaining specific measurement frequencies within the subrange of the frequency range or within the complete frequency range.

According to one embodiment of the present invention, the processor is further configured apply the second optimized frequency range for quality control measurements of the polymer product.

According to one embodiment of the present invention, the processor is further configured to apply the second optimized frequency range for a currently employed melt flow index measurement.

A computer program performing the method of the present invention may be stored on a computer-readable medium. A computer-readable medium may be a floppy disk, a hard disk, a CD, a DVD, an USB (Universal Serial Bus) storage device, a RAM (Random Access Memory), a ROM (Read Only Memory) and an EPROM (Erasable Programmable Read Only Memory).

A computer-readable medium may also be a data communication network, for example the Internet, which allows downloading a program code, with a connection via WLAN or 3G/4G or any other wireless data technology.

The methods, systems and devices described herein may be implemented as software in a Digital Signal Processor, DSP, in a micro-controller or in any other side-processor or as hardware circuit within an application specific integrated circuit, ASIC, CPLD or FPGA.

The present invention can be implemented in digital electronic circuitry, or in computer hardware, firmware, software, or in combinations thereof, e.g. in available hardware of conventional mobile devices or in new hardware dedicated for processing the methods described herein.

### BRIEF DESCRIPTION OF THE DRAWINGS

A more complete appreciation of the invention and the attendant advantages thereof will be more clearly understood by reference to the following schematic drawings, which are not to scale, wherein:
Fig. 1 shows a schematic diagram of a method for adapting operation settings for a frequency sweep measurement of a polymer product according to an exemplary embodiment of the invention; and
Fig. 2 shows a schematic diagram of an apparatus for adapting operation settings for a frequency sweep measurement of a polymer product according to an exemplary embodiment of the invention.

### DETAILED DESCRIPTION OF EMBODIMENTS

The illustration in the drawings is schematically and not to scale. In different drawings, similar or identical elements are provided with the same reference numerals.

Generally, identical parts, units, entities or steps are provided with the same reference symbols in the figures.

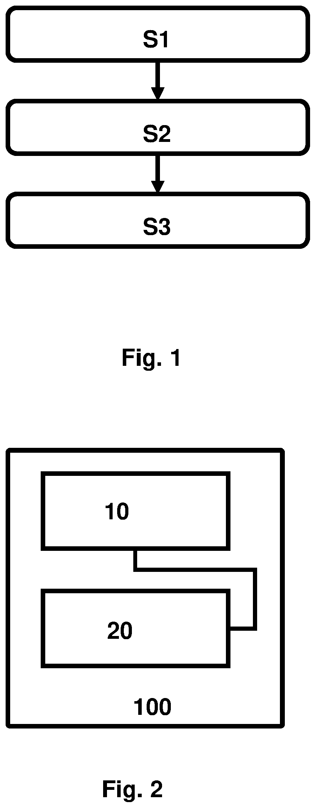

Fig. 1 shows a schematic diagram of a method for adapting operation settings for a frequency sweep measurement of a polymer product according to an exemplary embodiment of the invention.

In particular, Fig. 1 shows a method for adapting operation settings for a frequency sweep measurement of a polymer product, the method comprising the following steps.

As a first step of the method, providing S1 at least one set of operation setting parameters comprising at least one frequency range for the frequency sweep measurement of the polymer product, the at least one frequency range comprising a plurality of specific measurement frequencies is conducted.

As a second step of the method, optimizing S2 the frequency range by deleting at least one measurement frequency of the plurality of specific measurement frequencies within a lower half of the frequency range resulting in a first optimized frequency range is performed.

As a third step of the method, optimizing S3 the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range is performed.

The method for adapting operation settings for a frequency sweep measurement of a polymer product method refers to special operation settings for the frequency sweep measurement that leads to significant reduction of measurement time while the method accuracy remains at an adequate level for the needs of plant control via fast frequency sweep. The temperature is the same for both standard and special operation settings.

According to an exemplary embodiment of the present invention, for polyethylene, the test temperature is set to, for example, 190°C which is the same as for the MFI measurement.

According to an exemplary embodiment of the present invention, two method parameters which contribute in reducing the measurement time have been varied:
i. Frequency range (or equivalent shear rate) - Low frequency measurements are the slowest (measurement point time is proportional to inverse frequency). Removing the low frequency measurements, leads to significant time reduction. The frequency range is narrowed down from, for example, 628-0.01 rad/s to 250-0.1 rad/s.
ii. Number of measurement points per decade - This corresponds to the amount of measurement points measured per 'decade' (referring to the logarithmic scale) within the intended frequency range. This parameter has been changed from for example, 5 to 3 point/ decade.

According to an exemplary embodiment of the present invention, settings lead to significant reduction of the measurement time. Indicatively, the frequency sweep sample preparation and measurement time varies between 60 to 120 minutes, depending on the sample rheological characteristics, i.e. high viscosity polymers require higher measurement times. The use of the above settings reduces the measurement time to a range of 10 to 15 minutes.

The method for adapting operation settings for a frequency sweep measurement of a polymer product refers offers an attractive method for use in the plant environment:
The Fast frequency sweep is able to provide the experimental results faster. The sample preparation method is comparable to the currently employed melt index method.

The method equipment complexity and the technical competence required are at the same level with MFI method.

The method for adapting operation settings for a frequency sweep measurement of a polymer product provides a more complete rheological insight on the complex shear viscosity curve compared to MFI that corresponds to a single point of the curve (at an unknown shear rate) and the acquired complex viscosity curve is a unique fingerprint for the characterized polymer, reflecting its microstructure in terms of MWD and comonomer content.

On the contrary, MFI may be misleading since different MWD may lead to the same MFI value.

The reduced shear rate range of the method still provides adequate feedback information for the scope of plant monitoring and control.

Fig. 2 shows a schematic diagram of an apparatus for adapting operation settings for a frequency sweep measurement of a polymer product according to an exemplary embodiment of the invention.

An apparatus 100 apparatus for adapting operation settings for a frequency sweep measurement of a polymer product is provided, the apparatus 100 comprises data memory 10 and a processor 20.

The data memory 10 is configured to provide at least one set of operation setting parameters comprising at least one frequency range for the frequency sweep measurement of the polymer product, the at least one frequency range comprising a plurality of specific measurement frequencies.

The processor 20 is configured to optimize the frequency range by deleting at least one measurement frequency of the plurality of specific measurement frequencies within a lower half of the frequency range resulting in a first optimized frequency range.

The processor 20 is configured to optimize the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range.

The processor 20 may be implemented as Digital Signal Processor, DSP, in a micro-controller or in any other side-processor or as hardware circuit within an application specific integrated circuit, ASIC, CPLD or FPGA.

The processor 20 may be implemented as a Field Programmable Gate Arrays in terms of an integrated circuit that contains large numbers of identical logic cells.

The continuous need for advanced characterization methods for plant quality monitoring and control is well established. A QC method able to exceed MFI capabilities but still able to be used in plant environment will enable better plant monitoring and control level to current polymer plants.

Furthermore, a more efficient detailed kinetics, molecular properties-oriented model predictive control model has been developed. The new modelling features are able to monitor and control the polymer molecular properties and further estimate the corresponding rheological properties. Model predictive control is a powerful process tool, nevertheless, constant process feedback is needed in order to ensure the validity of the predictions.

For the new control concept, melt index values are useful but not enough - MWD or complex viscosity over large shear rate range feedback is required. This way, the limited control feedback becomes a barrier for model predictive control steps.

It has to be noted that embodiments of the invention are described with reference to different subject matters. In particular, some embodiments are described with reference to method type claims whereas other embodiments are described with reference to the device type claims.

However, a person skilled in the art will gather from the above and the following description that, unless otherwise notified, in addition to any combination of features belonging to one type of subject matter also any combination between features relating to different subject matters is considered to be disclosed with this application.

In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A computer-implemented method for adapting operation settings for a frequency sweep measurement of a polymer product, the method comprising the steps of:
- providing (S1) at least one set of operation setting parameters comprising at least one frequency range for the frequency sweep measurement of the polymer product, the at least one frequency range comprising a plurality of specific measurement frequencies;
- optimizing (S2) the frequency range by deleting at least one measurement frequency of the plurality of specific measurement frequencies within a lower half of the frequency range resulting in a first optimized frequency range; and
- optimizing (S3) the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range.

2. The method according to claim 1,
wherein after the step of optimizing (S2) the frequency range by deleting at least one measurement frequency, the first optimized frequency range is further optimized by equally distributing the remaining specific measurement frequencies within the lower half of the frequency range or within the complete frequency range.

3. The method according to claim 1 or 2,
wherein after the step of optimizing (S3) the first optimized frequency range, the second optimized frequency range is further optimized by equally distributing the remaining specific measurement frequencies within the subrange of the frequency range or within the complete frequency range.

4. The method according to any one of the preceding claims,
wherein the method further comprises the step of validating (S4) a consistency of the second optimized frequency range by comparing a calculated complex viscosity value to a measured melt flow index value using the second optimized frequency range.

5. The method according to any one of the preceding claims,
wherein the method further comprises the steps of
applying (S5) the second optimized frequency range for quality control measurements of the polymer product; and/or
applying (S6) the second optimized frequency range for a currently employed melt flow index measurement.

6. The method according to any one of the preceding claims,
wherein the method further comprises the step of generating (S7) the second optimized frequency range for a specific polymer grade of interest of the polymer product.

7. The method according to claim 6,
wherein by generating (S7) the second optimized frequency range for the specific polymer grade of interest of the polymer product, a frequency sweep fingerprint for the specific polymer grade is defined.

8. The method according to any one of the preceding claims,
wherein the set of operation setting parameters comprises at least one shear rate.

9. An apparatus (100) for adapting operation settings for a frequency sweep measurement of a polymer product, the apparatus comprising
- a data memory (10) which is configured to provide at least one set of operation setting parameters comprising at least one frequency range for the frequency sweep measurement of the polymer product, the at least one frequency range comprising a plurality of specific measurement frequencies; and
- a processor (20), which is configured to optimize the frequency range by deleting at least one measurement frequency of the plurality of specific measurement frequencies within a lower half of the frequency range resulting in a first optimized frequency range;
wherein the processor (20) is further configured to optimize the first optimized frequency range by determining a value of measurement frequencies present per decade of frequency within at least a subrange of the frequency range and reducing the determined value of measurement frequencies present per decade within the at least one subrange of the frequency range by deleting at least one measurement frequency of the at least one subrange resulting in a second optimized frequency range.

10. The apparatus according to claim 9,
wherein the processor (20) is further configured to optimize the first optimized frequency range by equally distributing the remaining specific measurement frequencies within the lower half of the frequency range or within the complete frequency range.

11. The apparatus according to claim 9 or 10,
wherein the processor (20) is further configured to optimize the second optimized frequency range by equally distributing the remaining specific measurement frequencies within the subrange of the frequency range or within the complete frequency range.

12. The apparatus according to any one of the preceding claims 9 to 11,
wherein the processor (20) is further configured to validate a consistency of the second optimized frequency range by comparing a calculated complex viscosity value to a measured melt flow index value using the second optimized frequency range.

13. The apparatus according to any one of the preceding claims 9 to 12,
wherein the processor (20) is further configured to generate the second optimized frequency range for a specific polymer grade of interest of the polymer product.

14. The apparatus according to any one of the preceding claims 9 to 13,
wherein the processor (20) is further configured apply the second optimized frequency range for quality control measurements of the polymer product; and/or
wherein the processor (20) is further configured to apply the second optimized frequency range for a currently employed melt flow index measurement.

15. A computer-readable medium comprising instructions which, when executed by a computer, cause the computer to carry out the steps of the method of any one of the preceding claims 1 to 8.

## Patentansprüche

1. Computer-implementiertes Verfahren zum Anpassen von Betriebseinstellungen für eine Frequenz-Sweep-Messung eines Polymerprodukts, wobei das Verfahren die folgenden Schritte aufweist:
- Bereitstellen (S1) mindestens eines Satzes von Betriebseinstellungsparametern, der mindestens einen Frequenzbereich für die Frequenz-Sweep-Messung des Polymerprodukts aufweist, wobei der mindestens eine Frequenzbereich eine Vielzahl von spezifischen Messfrequenzen umfasst;
- Optimieren (S2) des Frequenzbereichs durch Streichen mindestens einer Messfrequenz der Vielzahl spezifischer Messfrequenzen innerhalb einer unteren Hälfte des Frequenzbereichs, was zu einem ersten optimierten Frequenzbereich führt; und
- Optimieren (S3) des ersten optimierten Frequenzbereichs durch Bestimmen eines Werts von Messfrequenzen, die pro Frequenzdekade innerhalb mindestens eines Unterbereichs des Frequenzbereichs vorhanden sind, und Reduzieren des bestimmten Werts von Messfrequenzen, die pro Dekade innerhalb des mindestens einen Unterbereichs des Frequenzbereichs vorhanden sind, durch Streichen mindestens einer Messfrequenz des mindestens einen Unterbereichs, was zu einem zweiten optimierten Frequenzbereich führt.

2. Verfahren nach Anspruch 1, wobei nach dem Schritt des Optimierens (S2) des Frequenzbereichs durch Streichen mindestens einer Messfrequenz, der erste optimierte Frequenzbereich durch gleichmäßiges Verteilen der verbleibenden spezifischen Messfrequenzen innerhalb der unteren Hälfte des Frequenzbereichs oder innerhalb des gesamten Frequenzbereichs weiter optimiert wird.

3. Verfahren nach Anspruch 1 oder 2, wobei nach dem Schritt des Optimierens (S3) des ersten optimierten Frequenzbereichs, der zweite optimierte Frequenzbereich durch gleichmäßiges Verteilen der verbleibenden spezifischen Messfrequenzen innerhalb des Unterbereichs des Frequenzbereichs oder innerhalb des gesamten Frequenzbereichs weiter optimiert wird.

4. Verfahren nach einem der vorhergehenden Ansprüche, wobei das Verfahren ferner den Schritt der Validierung (S4) einer Konsistenz des zweiten optimierten Frequenzbereichs durch Vergleich eines berechneten komplexen Viskositätswerts mit einem gemessenen Schmelzflussindexwert unter Verwendung des zweiten optimierten Frequenzbereichs aufweist.

5. Verfahren nach einem der vorhergehenden Ansprüche, wobei das Verfahren ferner die Schritte der Anwendung (S5) des zweiten optimierten Frequenzbereichs für Qualitätskontrollmessungen des Polymerprodukts und/oder der Anwendung (S6) des zweiten optimierten Frequenzbereichs für eine gegenwärtig verwendete Schmelzflussindexmessung aufweist.

6. Verfahren nach einem der vorhergehenden Ansprüche, wobei das Verfahren ferner den Schritt des Erzeugens (S7) des zweiten optimierten Frequenzbereichs für eine spezifische Polymersorte von Interesse des Polymerprodukts aufweist.

7. Verfahren nach Anspruch 6, wobei durch Erzeugen (S7) des zweiten optimierten Frequenzbereichs für die spezifische Polymersorte von Interesse des Polymerprodukts ein Frequenz-Sweep-Fingerabdruck für die spezifische Polymersorte definiert ist.

8. Verfahren nach einem der vorhergehenden Ansprüche, wobei der Satz der Betriebseinstellungsparameter mindestens eine Scherrate aufweist.

9. Vorrichtung (100) zum Anpassen von Betriebseinstellungen für eine Frequenz-Sweep-Messung eines Polymerprodukts, wobei die Vorrichtung aufweist
- einen Datenspeicher (10), der derart eingerichtet ist, dass er mindestens einen Satz von Betriebseinstellungsparametern bereitstellt, der mindestens einen Frequenzbereich für die Frequenz-Sweep-Messung des Polymerprodukts aufweist, wobei der mindestens eine Frequenzbereich eine Vielzahl von spezifischen Messfrequenzen aufweist; und
- einen Prozessor (20), der derart eingerichtet ist, dass er den Frequenzbereich durch Streichen mindestens einer Messfrequenz der Vielzahl spezifischer Messfrequenzen innerhalb einer unteren Hälfte des Frequenzbereichs optimiert, was zu einem ersten optimierten Frequenzbereich führt,; wobei der Prozessor (20) ferner derart eingerichtet ist, dass er den ersten optimierten Frequenzbereich durch Bestimmen eines Werts von Messfrequenzen, die pro Frequenzdekade innerhalb mindestens eines Unterbereichs des Frequenzbereichs vorhanden sind, und Reduzieren des bestimmten Werts von Messfrequenzen, die pro Dekade innerhalb des mindestens einen Unterbereichs des Frequenzbereichs vorhanden sind, durch Streichen mindestens einer Messfrequenz des mindestens einen Unterbereichs, was zu einem zweiten optimierten Frequenzbereich führt.

10. Vorrichtung nach Anspruch 9, wobei der Prozessor (20) ferner derart eingerichtet ist, dass er den ersten optimierten Frequenzbereich durch gleichmäßiges Verteilen der verbleibenden spezifischen Messfrequenzen innerhalb der unteren Hälfte des Frequenzbereichs oder innerhalb des gesamten Frequenzbereichs optimiert.

11. Vorrichtung nach Anspruch 9 oder 10, wobei der Prozessor (20) ferner derart eingerichtet ist, dass er den zweiten optimierten Frequenzbereich durch gleichmäßiges Verteilen der verbleibenden spezifischen Messfrequenzen innerhalb des Unterbereichs des Frequenzbereichs oder innerhalb des gesamten Frequenzbereichs optimiert.

12. Vorrichtung nach einem der vorangehenden Ansprüche 9 bis 11, wobei der Prozessor (20) ferner derart eingerichtet ist, dass er eine Konsistenz des zweiten optimierten Frequenzbereichs durch Vergleich eines berechneten komplexen Viskositätswerts mit einem gemessenen Schmelzflussindexwert unter Verwendung des zweiten optimierten Frequenzbereichs validiert.

13. Vorrichtung nach einem der vorangehenden Ansprüche 9 bis 12, wobei der Prozessor (20) ferner derart eingerichtet ist, dass er den zweiten optimierten Frequenzbereich für eine spezifische Polymersorte von Interesse des Polymerprodukts erzeugt.

14. Vorrichtung nach einem der vorhergehenden Ansprüche 9 bis 13, wobei der Prozessor (20) ferner derart eingerichtet ist, dass er den zweiten optimierten Frequenzbereich für Qualitätskontrollmessungen des Polymerprodukts anwendet; und/oder wobei der Prozessor (20) ferner derart eingerichtet ist, dass er den zweiten optimierten Frequenzbereich für eine gegenwärtig verwendete Schmelzflussindexmessung anwendet.

15. Computerlesbares Medium, umfassend Befehle, die bei der Ausführung durch einen Computer diesen veranlassen, die Schritte des Verfahrens nach einem der vorhergehenden Ansprüche 1 bis 8 auszuführen.

## Revendications

1. Procédé mis en œuvre par ordinateur pour adapter des réglages de fonctionnement pour une mesure de balayage de fréquence d'un produit polymère, le procédé comprenant les étapes consistant à :
- fournir (S1) au moins un jeu de paramètres de réglage de fonctionnement comprenant au moins une plage de fréquences pour la mesure de balayage de fréquence du produit polymère, l'au moins une plage de fréquences comprenant une pluralité de fréquences de mesure spécifiques ;
- optimiser (S2) la plage de fréquences en supprimant au moins une fréquence de mesure de la pluralité de fréquences de mesure spécifiques dans une moitié inférieure de la plage de fréquences, pour obtenir une première plage de fréquences optimisée ; et
- optimiser (S3) la première plage de fréquences optimisée en déterminant une valeur de fréquences de mesure présente par décade de fréquence dans au moins une sous-plage de la plage de fréquences et en réduisant la valeur déterminée de fréquences de mesure présente par décade dans l'au moins une sous-plage de la plage de fréquences par la suppression d'au moins une fréquence de mesure de l'au moins une sous-plage, pour obtenir une deuxième plage de fréquences optimisée.

2. Procédé selon la revendication 1,
dans lequel, après l'étape d'optimisation (S2) de la plage de fréquences par la suppression d'au moins une fréquence de mesure, la première plage de fréquences optimisée est encore optimisée en répartissant de manière égale les fréquences de mesure spécifiques restantes dans la moitié inférieure de la plage de fréquences ou dans la plage de fréquences complète.

3. Procédé selon la revendication 1 ou 2, dans lequel, après l'étape d'optimisation (S3) de la première plage de fréquences optimisée, la deuxième plage de fréquences optimisée est encore optimisée en répartissant de manière égale les fréquences de mesure spécifiques restantes dans la sous-plage de la plage de fréquences ou dans la plage de fréquences complète.

4. Procédé selon l'une des revendications précédentes,
dans lequel le procédé comprend en outre l'étape de validation (S4) d'une cohérence de la deuxième plage de fréquences optimisée en comparant une valeur de viscosité complexe calculée à une valeur d'indice de fluidité à chaud mesurée en utilisant la deuxième plage de fréquences optimisée.

5. Procédé selon l'une des revendications précédentes, dans lequel le procédé comprend en outre les étapes consistant à
appliquer (S5) la deuxième plage de fréquences optimisée pour des mesures de contrôle qualité du produit polymère ; et/ou
appliquer (S6) la deuxième plage de fréquences optimisée pour une mesure d'indice de fluidité à chaud actuellement utilisée.

6. Procédé selon l'une des revendications précédentes,
dans lequel le procédé comprend en outre l'étape de génération (S7) de la deuxième plage de fréquences optimisée pour une qualité d'intérêt de polymère spécifique du produit polymère.

7. Procédé selon la revendication 6,
dans lequel, en générant (S7) la deuxième plage de fréquences optimisée pour la qualité d'intérêt de polymère spécifique du produit polymère, une empreinte digitale de balayage de fréquence pour la qualité de polymère spécifique est définie.

8. Procédé selon l'une des revendications précédentes,
dans lequel le jeu de paramètres de réglage de fonctionnement comprend au moins un taux de cisaillement.

9. Appareil (100) pour adapter des réglages de fonctionnement pour une mesure de balayage de fréquence d'un produit polymère, l'appareil comprenant
- une mémoire de données (10) qui est configurée pour fournir au moins un jeu de paramètres de réglage de fonctionnement comprenant au moins une plage de fréquences pour la mesure de balayage de fréquence du produit polymère, l'au moins une plage de fréquences comprenant une pluralité de fréquences de mesure spécifiques ; et
- un processeur (20) qui est configuré pour optimiser la plage de fréquences en supprimant au moins une fréquence de mesure de la pluralité de fréquences de mesure spécifiques dans une moitié inférieure de la plage de fréquences, pour obtenir une première plage de fréquences optimisée ;
dans lequel le processeur (20) est configuré en outre pour optimiser la première plage de fréquences optimisée en déterminant une valeur de fréquences de mesure présente par décade de fréquence dans au moins une sous-plage de la plage de fréquences et en réduisant la valeur déterminée de fréquences de mesure présente par décade dans l'au moins une sous-plage de la plage de fréquences par la suppression d'au moins une fréquence de mesure de l'au moins une sous-plage, pour obtenir une deuxième plage de fréquences optimisée.

10. Appareil selon la revendication 9,
dans lequel le processeur (20) est configuré en outre pour optimiser la première plage de fréquences optimisée en répartissant de manière égale les fréquences de mesure spécifiques restantes dans la moitié inférieure de la plage de fréquences ou dans la plage de fréquences complète.

11. Appareil selon la revendication 9 ou 10,
dans lequel le processeur (20) est configuré en outre pour optimiser la deuxième plage de fréquences optimisée en répartissant de manière égale les fréquences de mesure spécifiques restantes dans la sous-plage de la plage de fréquences ou dans la plage de fréquences complète.

12. Appareil selon l'une des revendications 9 à 11 précédentes,
dans lequel le processeur (20) est configuré en outre pour valider une cohérence de la deuxième plage de fréquences optimisée en comparant une valeur de viscosité complexe calculée à une valeur d'indice de fluidité à chaud mesurée en utilisant la deuxième plage de fréquences optimisée.

13. Appareil selon l'une des revendications 9 à 12 précédentes,
dans lequel le processeur (20) est configuré en outre pour générer la deuxième plage de fréquences optimisée pour une qualité d'intérêt de polymère spécifique du produit polymère.

14. Appareil selon l'une des revendications 9 à 13 précédentes,
dans lequel le processeur (20) est configuré en outre pour appliquer la deuxième plage de fréquences optimisée pour des mesures de contrôle qualité du produit polymère ; et/ou
dans lequel le processeur (20) est configuré en outre pour appliquer la deuxième plage de fréquences optimisée pour une mesure d'indice de fluidité à chaud actuellement utilisée.

15. Support lisible par ordinateur comprenant des instructions qui, lorsqu'elles sont exécutées par un ordinateur, amènent l'ordinateur à exécuter les étapes du procédé selon l'une des revendications 1 à 8 précédentes.
